# EUROPÄISCHE PATENTSCHRIFT

(11) **EP 0 999 230 B1**
(45) Veröffentlichungstag und Bekanntmachung des Hinweises auf die Patenterteilung: **16.04.2003**
(21) Anmeldenummer: 99121019.6
(22) Anmeldetag: 21.10.1999
(51) Int. Cl.: C08G 77/08, C08G 77/38, C08G 77/42, C08G 77/46

(54) **Verfahren zur Herstellung von Acrylsäureestern und/oder Methacrylsäureestern von hydroxyfunktionellen Siloxanen und/oder polyoxyalkylenmodifizierten Siloxanen und deren Verwendung**
Method for preparing (meth)acrylic acid esters of hydroxy or polyoxyalkylene modified siloxanes and their use
Méthode pour la préparation des esters d'acide (meth)acrylique et siloxanes à fonctions hydroxy ou polyoxyalkylène et leur usage

(30) Priorität: 03.11.1998 DE 19850507
(43) Veröffentlichungstag der Anmeldung: 10.05.2000
(73) Patentinhaber: Goldschmidt AG, 45127 Essen (DE)
(72) Erfinder: Grüning, Burghard, Dr., 45134 Essen (DE); Hills, Geoffrey, 45355 Essen (DE); Josten, Wolfgang, Dr., 53639 Königswinter (DE); Schaefer, Dietmar, Dr., 45529 Hattingen (DE); Silber, Stefan, Dr., 47804 Krefeld (DE); Weitemeyer, Christian, Dr., 45134 Essen (DE)

(56) Entgegenhaltungen:
- DE-A- 4 408 152
- Enzyme and microbial technology, 1990, vol 12, April, pp 299-304, "Enzymatically catalysed transesterifications of acryl and methacryl monomeric esters", Tor et al. *abstract* XP002138281

## Beschreibung

Gegenstand der vorliegenden Erfindung ist ein Verfahren zur Ver- oder Umesterung von Acrylsäure und/oder Methacrylsäure oder Acrylsäureestern und/oder Methacrylsäureestern in Anwesenheit eines die Ver- oder Umesterung katalysierenden Enzyms mit hydroxyfunktionellen und/oder polyoxyalkylenmodifizierten Siloxanderivaten sowie deren Verwendung.

Unter den Rohstoffen zur Herstellung von Polymerprodukten hat die Verarbeitung von Acrylmonomeren in den letzten Jahren eine stürmische Entwicklung genommen. Acrylmonomere finden überwiegend Einsatz bei der Herstellung von Fasern, Dispersionen, Lackrohstoffen, Klebrohstoffen und thermoplastischen Massen. Geringere Mengen dienen als Ausgangsstoffe für unterschiedliche chemische Synthesen.

Dementsprechend gibt es eine Vielzahl von Verfahren zur Herstellung entsprechender Acrylate und/oder Methacrylate. Im Sinne dieser Erfindung ist unter "Acryl" oder "Methacryl" ein Radikal der allgemeinen Formel 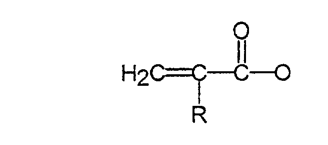 zu verstehen, wobei R = CH₃ oder H ist.

Neben allgemein üblichen Verfahren zur Herstellung von Acrylaten und/oder Methacrylaten, die im wesentlichen literaturbekannten Herstellungsverfahren für Carbonsäureester entsprechen (Übersicht in J. March, Advanced Organic Chemistry, Wiley, 1992), sind im Zusammenhang mit der Modifizierung von hydroxyfunktionellen Siloxanen und/oder polyoxyalkylenmodifizierten Siloxanen mit Acryl- und/oder Methacrylsäureestern oder Acryl- und/oder Methacrylsäure auch speziell beschriebene Verfahren bekannt.

Die gängigen Verfahren gehen dabei von hydroxyfunktionellen Vorstufen aus und führen die Acryl- und/oder Methacryloylgruppe durch Veresterungs- oder Umesterungsverfahren, ausgehend von den entsprechenden Acryl- und/oder Methacrylsäuren oder Acryl- und/oder Methacrylsäureestern, ein. Im Regelfall ist dazu die Anwesenheit von Katalysatoren unerläßlich. In US-A-4 777 265 werden beispielsweise Chelatkomplexe von Titan, Zirkon, Eisen und Zink mit 1,3-Dicarbonylverbindungen als Katalysatoren für solche Umsetzungen beschrieben. Häufig dienen auch Säuren zur Katalyse einer entsprechenden Veresterungsreaktion, so beispielsweise in US-A-5 091 440.

Diese Verfahren laufen im Regelfall bei Temperaturen von oberhalb von 80 °C, häufig oberhalb 100 °C ab und bedürfen einer zusätzlichen Stabilisierung des Reaktionsgemisches durch Radikalfänger (zum Beispiel Methylhydrochinon), um eine unerwünschte Polymerisation der Acrylate und/oder Methacrylate bei diesen Temperaturen verläßlich zu unterdrücken. Der Katalysator muß anschließend für viele Anwendungsgebiete entfernt oder aber zumindest neutralisiert werden, um unerwünschte Folgereaktionen zu vermeiden. Dies erfordert einen aufwendigen Aufarbeitungsprozeß, indem man Metalloxide, Metallhydroxide oder entsprechende Salze der Metalle beziehungsweise der als Katalysator verwendeten Säuren bildet und anschließend im Regelfall abfiltriert. Derartige Filtrationen acryl- und/oder methacryloylhaltiger Reaktionsgemische sind technologisch und arbeitssicherheitstechnisch aufwendig und dadurch oftmals langwierig. Bedingt durch die hohe Reaktionstemperatur sind so hergestellte acryl- und/oder methacryloylfunktionelle Verbindungen häufig intensiv gefärbt (gelb bis braun-schwarz). Dies verhindert oft eine direkte Verwendung solcher Acrylund/oder Methacryloylverbindungen in Anwendungen mit erhöhten Anforderungen mit Bezug auf die Färbung der eingesetzten Rohstoffe. Hier ist beispielsweise die Verwendung als Additiv in strahlenhärtenden Beschichtungen, insbesondere Klarlacken, zu nennen.

Auch die direkte Umsetzung von Alkoxy-, Hydroxyl- oder Chlorsiloxanen mit hydroxyfunktionellen Acrylaten und/oder Methacrylaten ist in DE-A-27 47 233 beschrieben. Auch hier sind Metallkatalysatoren, erhöhte Temperaturen oberhalb 120 °C und zusätzliche Inhibitoren notwendig. Somit wohnen auch diesem Verfahren die obengenannten Nachteile mit Bezug auf hohe Farbzahlen, Katalysatorrestgehalte und unerwünschter Polymerisation der Acryl- und/oder Methacrylatgruppen inne.

Daneben gibt es auch Verfahren, die sich einer wenigstens difunktionellen Verknüpfungseinheit bedienen durch die beispielsweise, wie in US-A-4 218 294, US-A-4 369 300, US-A-4 130 708, US-A-4 369 300, EP-A-0 518 020, WO 86/02652 oder DE-A-30 44 301 beschrieben., eine hydroxyfunktionelle Verbindung mit einem hydroxyfunktionellen Acrylat (zum Beispiel Hydroxyethylacrylat) durch Reaktion mit einem Diisocyanat (zum Beispiel Isophorondiisocyanat) unter Ausbildung wenigstens zweier Urethanbrücken miteinander verknüpft wird. Gleichermaßen werden auch kommerziell erhältliche Methacryl- und/oder Acrylatmonomere angeboten, die noch eine Isocyanatgruppe im Molekül tragen (zum Beispiel TMI®, meta-Isoprenyl-α,α-Dimethylbenzylisocyanat; Fa. Cyanamid) und somit auch zur Reaktion mit Alkoholen befähigt sind. Auch von diesen toxikologisch bedenklichen Grundbausteinen ausgehend bildet sich eine Urethangruppe, die die beiden Strukturelemente miteinander verknüpft. Die Reaktion zwischen Isocyanat- und Hydroxygruppe muß im Regelfall durch die Zugabe von Katalysatoren (zum Beispiel Zinnverbindungen oder Amine) katalysiert werden. Diese Katalysatoren verbleiben dann im Endprodukt. Weitere Verknüpfungsreaktionen beispielsweise über Oxiranderivate sind zum Beispiel in DE-A-39 32 460, J. Appl. Polym. Sc., Vol. 59, 1937 - 1944 beschrieben.

Diesen Verfahren, die zusätzliche funktionelle Gruppen in die entstehenden Acryl- und/oder Methacryloylverbindungen einbringen (beispielsweise Urethane, Harnstoffe, β-Hydroxyester, Ester etc.), haftet der praktische Nachteil an, daß diese zusätzlichen Strukturelemente einen limitierenden Einfluß auf mögliche weitere Modifizierungen dieser Verbindungen zum Beispiel durch Nebenreaktionen ausüben. Außerdem können auch Verarbeitungsparameter, wie zum Beispiel die Viskosität, unerwünscht beeinflußt werden. Teilweise wird auch die Struktur der so hergestellten organomodifizierten Siloxanmethacrylate und/oder Siloxanacrylate so beeinflußt, daß erwünschte oder unerwünschte grenzflächenaktive Eigenschaften verändert werden und somit systematische Entwicklungsarbeiten zusätzlich erschwert werden.

R. Tor, Enzyme Micro. Technol., 1990, Vol. 12, April, S. 299 - 304, beschreibt die enzymatisch katalysierte Umesterung von Acryl- und Methacrylmonomerestern zur Herstellung von Hydroxy- und Dihydroxyalkylacrylaten und -methacrylaten ohne Bildung von Di- oder Triacrylat und -methacrylaten. Insbesondere werden 2-Hydroxyethyl-, 2-Hydroxypropyl- und 1,2-Dihydroxypropylester von Acrylsäure und Methacrylsäure untersucht.

Die Aufgabe der vorliegenden Erfindung besteht in der Bereitstellung eines vereinfachten Verfahrens zur Veresterung oder Umesterung von Acryl- und/oder Methacrylsäure oder Acryl- und/oder Methacrylsäureestern mit hydroxyfunktionellen Siloxanen und/oder polyoxyalkylenmodifizierten Siloxanen. Ein solches Herstellungsverfahren soll insbesondere eine deutlich hellere Farbe der Reaktionsprodukte ermöglichen, die Bildung von Nebenprodukten (aufgrund unselektiver Katalyse) vermeiden, eine unkomplizierte Entfernung des Enzymkatalysators vom Produkt ermöglichen und unerwünschte und unkontrollierte radikalische Polymerisationen der Acryl- und/oder Methacrylatderivate vermeiden.

Die vorstehend genannte Aufgabe wird gelöst durch ein Verfahren zur Ver- oder Umesterung von Acrylsäure und/oder Methacrylsäure oder Acrylsäureestern und/oder Methacrylsäureestern in Anwesenheit eines die Ver- oder Umesterung katalysierenden Enzyms mit hydroxyfunktionellen und/oder polyoxyalkylenmodifizierten Siloxanderivaten der allgemeinen Formel (I) 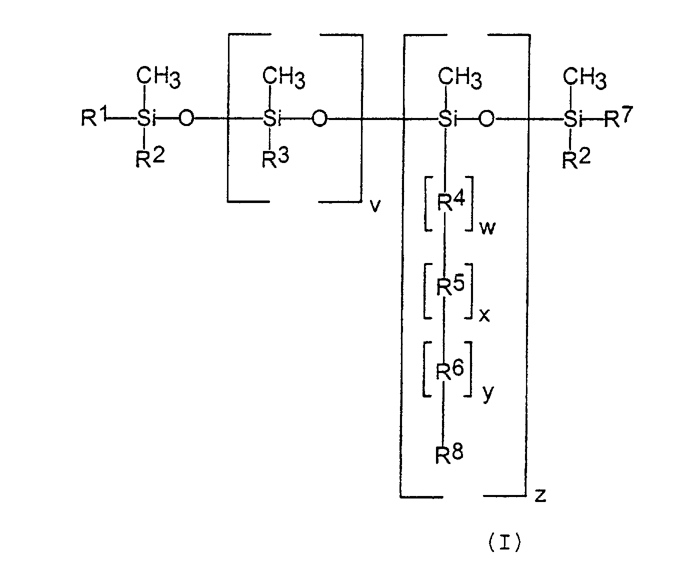 wobei
R¹ und/oder R⁷ = R² oder [R⁴]_{w}― [R⁵]ₓ― [R⁶]_{y}― R⁸,
R² = R³ oder ≠ R³ für gleiche oder verschiedene Alkylreste oder Alkylenreste mit 1 bis 24 Kohlenstoffatomen oder gegebenenfalls substituierte Phenylreste mit bis zu 24 Kohlenstoffatomen steht,
R⁴ = ein zweiwertiger Rest der Formel O, NH, NR², S oder ein Rest der Formel (OSi(CH₃)₂)ᵤ, wobei
u = 1 bis 200,
R⁵ = gleiche oder verschiedene Alkylreste oder Alkylenreste mit 1 bis 24 Kohlenstoffatomen, oder
CₙH_{2n-f}R²_{f}― R⁴― CₘH_{2m-g}R²_{g}, wobei
f = 0 bis 12,
g = 0 bis 12,
n = 1 bis 18,
m = 1 bis 18,
R⁶ = O- (C₂H₄₋ₐR²ₐO)_{b}(C_{c}H_{2c}O)_{d}, wobei
a = 0 bis 3,
b = 0 bis 100,
c = 2 bis 12,
d = 0 bis 100,
die Summe (b + d) = 1 bis 200 ist
und die Reihenfolge der einzelnen Polyoxyalkylensegmente (C₂H₄₋ₐR²ₐO)_{b} und (C_{c}H_{2c}O)_{d} beliebig sein kann und insbesondere Blockcopolymere, wie statistische Polymere sowie deren Kombinationen, umfaßt, oder
R⁶ = Oₑ-CₕH₂ₕ-CᵢH₂ᵢ₋ⱼR⁹ⱼ, wobei
e = 0 oder 1,
h = 0 bis 24,
i = 0 bis 24,
j = 1 bis 3,
die Summe (w + e) = 0 bis 1 ist
und R⁹ jeweils ein zweiwertiger Rest der Formel O, eine Hydroxygruppe, ein Rest der Formel CₕH₂ₕ oder ein Rest der Formel CₖH₂ₖ₋₁(OH)₁ bedeutet, wobei
k = 0 bis 24 und
1 = 1 bis 3 ist,
R⁸ = ein Wasserstoffrest oder ein einwertiger organischer Rest ist, wenn y gleich 1 ist, wobei pro Molekül mindestens ein Wasserstoffrest vorhanden sein muß, oder eine OH-Gruppe oder ein einwertiger organischer Rest, wenn y = = 0, wobei pro Molekül mindestens eine OH-Gruppe vorhanden ist,
v = 0 bis 200,
w = 0 oder 1,
x = 0 oder 1,
y = 0 oder 1,
z = 0 bis 200
und die Summe (w + x + y) = 1 bis 3
und wenn z = 0 ist, R¹ und/oder R⁷ gleich
[R⁴]_{w}― [R⁵]ₓ― [R⁶]_{y}― R⁸ ist
und wenn x = 0 dann auch w = 0 ist.

Es ist dem Fachmann geläufig, daß die Verbindungen in Form eines Gemisches mit einer im wesentlichen durch statistische Gesetze geregelten Verteilung vorliegen. Insbesondere die Werte für die Indices b, d, u, v und z stellen deshalb Mittelwerte dar.

Beispiele von Siloxanderivaten, die erfindungsgemäß durch enzymatisch katalysierte Ver- oder Umesterung von Acrylund/oder Methacrylsäure oder Acryl- und/oder Methacrylsäureestern umgesetzt werden können, sind:

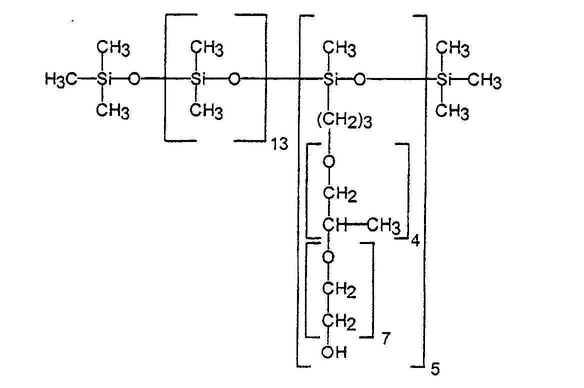

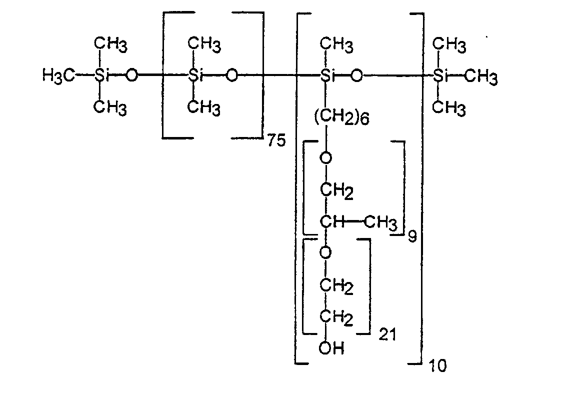

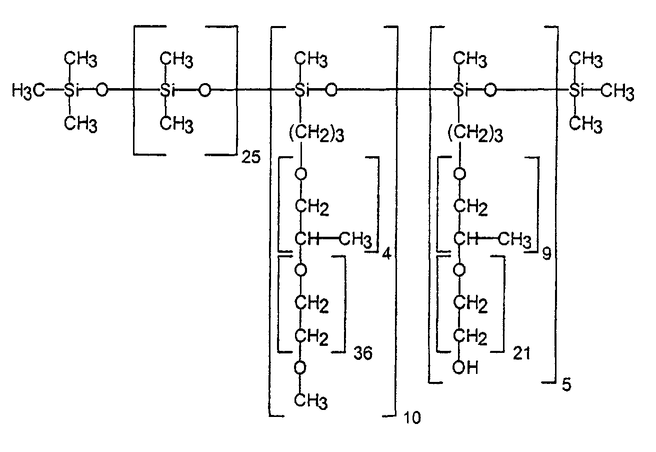

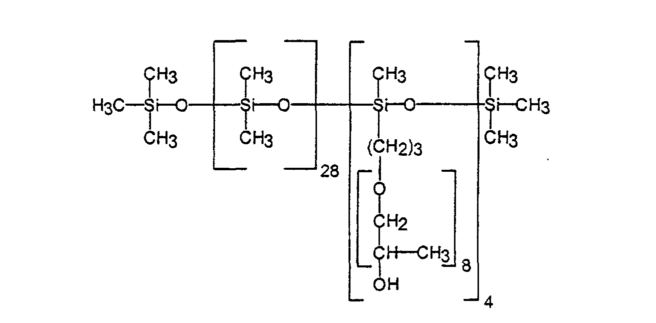

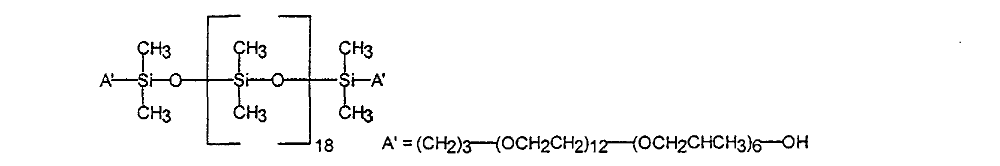

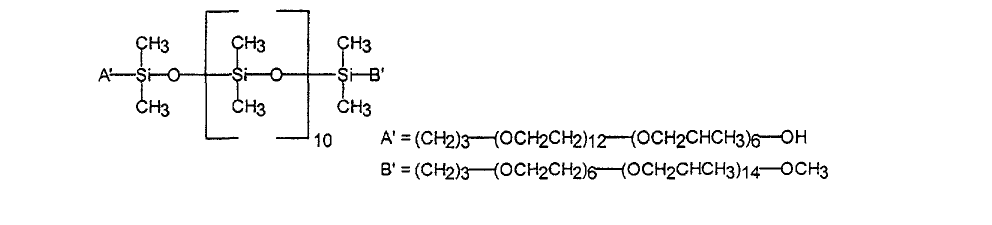

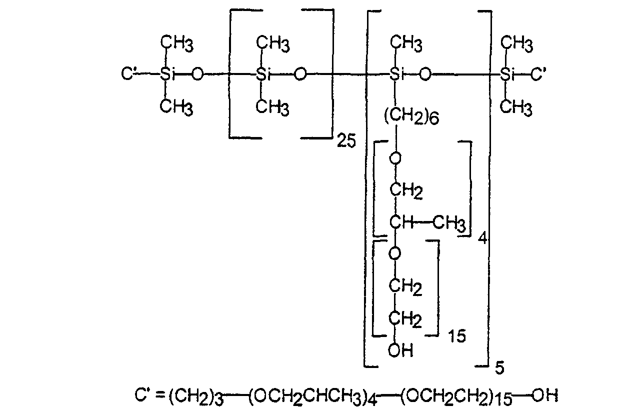

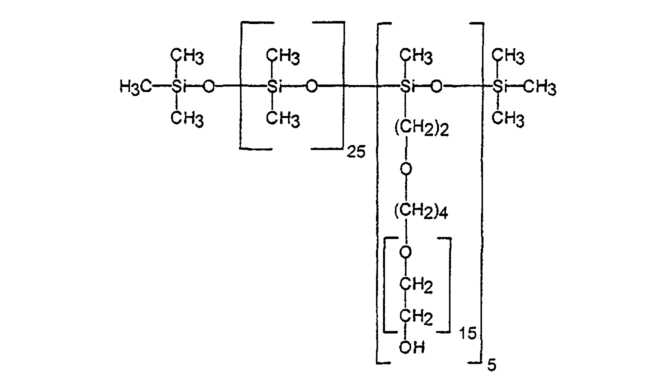

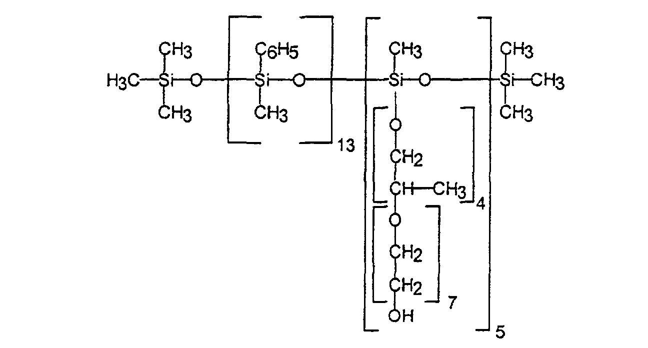

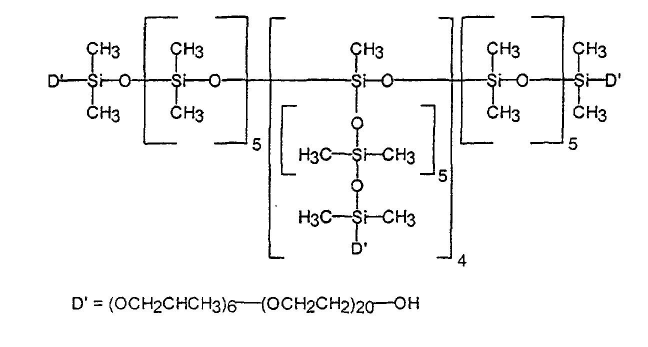

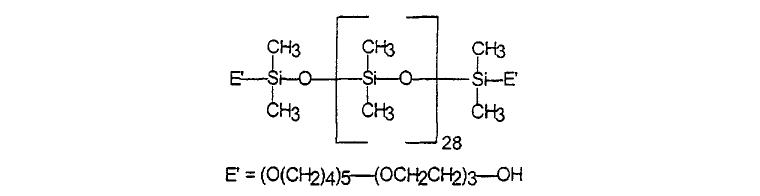

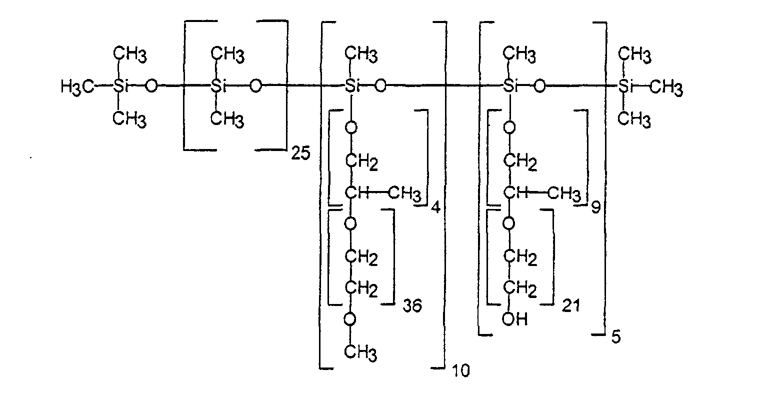

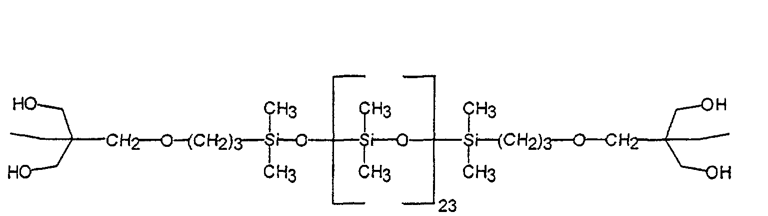

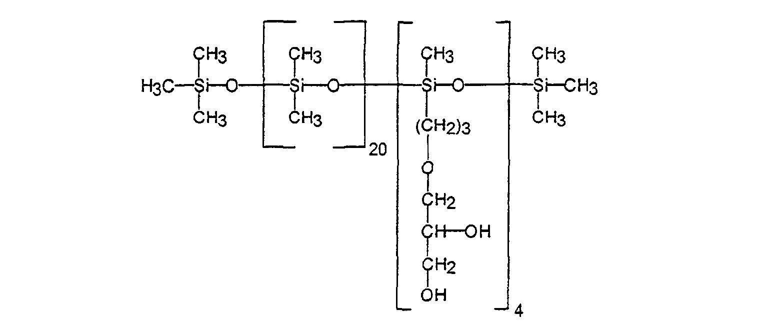

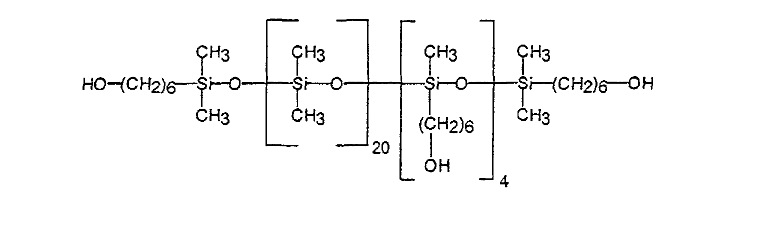

Die enzymatische Ver- oder Umesterung von Acryl- und/oder Methacrylsäure oder Acryl- und/oder Methacrylsäureestern mit den oben genannten Verbindungen bei niedrigen Temperaturen, insbesondere 20 bis 100 °C, bevorzugt 40 bis 70 °C und milden Bedingungen, ist vorteilhaft aufgrund der helleren Farbe des Produkts, der Vermeidung der Bildung von Nebenprodukten, die andernfalls zum Beispiel von chemischen Katalysatoren stammen können, der unkomplizierten Entfernung des Enzymkatalysators vom Produkt und der Vermeidung unerwünschter und unkontrollierter radikalischer Polymerisation der Acryl- und/oder Methacryloylverbindungen.

Die erfindungsgemäß erhältlichen acryloyl- und/oder methacryloylfunktionellen Siloxanderivate zeichnen sich dadurch aus, daß 5 bis 100 % aller ursprünglich vorhandenen Hydroxygruppen zu einem Acryl- und/oder Methacrylsäureester umgesetzt worden sind.

Die Acrylierung und/oder Methacrylierung verläuft am besten in hohen Ausbeuten mit Estern der Acryl- und/oder Methacrylsäure als Donormoleküle, insbesondere Methyl-, Ethyl- oder Butylmethacrylat und/oder -acrylat.

Enzyme, die bevorzugt als Katalysatoren eingesetzt werden können, sind Hydrolasen, insbesondere Esterasen, Lipasen und Proteasen. Die Enzyme können in reiner Form oder in immobilisierter Form auf einem Träger, auf dem sie chemisch oder physikalisch gebunden sind, eingesetzt werden. Die Menge des Enzymkatalysators beträgt insbesondere, bezogen auf das eingesetzte modifizierte Siloxan, 0,1 bis 20 Gew.-%, vorzugsweise 1 bis 10 Gew.-%. Die Reaktionszeit hängt von der verwendeten Menge und der Aktivität des Enzymkatalysators ab und beträgt beispielsweise bis zu 48 Stunden, vorzugsweise bis zu 24 Stunden.

Um unter einfachen Reaktionsbedingungen schnell zu hohen Umsetzungsgraden zu kommen, ist es vorteilhaft, einen Überschuß von wenigstens 10 Gew.-% Acrylsäure bzw. Methacrylsäure und/oder deren entsprechenden Estern (als Donoren) in der Reaktionsmischung zu verwenden.

Das Produktionssystem läßt sich entweder durch einen Rührkesselreaktor oder einen Festbettreaktor charakterisieren. Der Rührkesselreaktor kann mit einer Vorrichtung zum Abdestillieren des aus dem Acryl- und/oder Methacrylsäuredonor freigesetzten Alkanols beziehungsweise des aus der Acrylsäure und/oder Methacrylsäure freigesetzten Wassers ausgestattet sein.

Die Reaktion wird durchgeführt bis der gewünschte Umsatz erreicht wird. Eine Reaktionsführung mit gleichzeitiger Destillation wird bevorzugt, weil die Entfernung des Reaktionswassers bzw. Reaktionsalkanols zu höheren Umsätzen in kürzeren Reaktionszeiten aufgrund der Verschiebung des Reaktionsgleichgewichts führt.

Um möglichst hohe Umsetzungsgrade zu erreichen, ist die Entfernung des Reaktionswassers bzw. -alkanols notwendig.

Nach beendeter Umsetzung kann der Enzymkatalysator durch geeignete Maßnahmen, wie Filtrieren oder Dekantieren, abgetrennt werden und gegebenenfalls mehrmals eingesetzt werden. Der Festbettreaktor ist mit immobilisierten Enzymen bestückt, wobei die Reaktionsmischung durch die mit Katalysator gefüllte Säule gepumpt wird. Mit einem auf einem Träger immobilisierten Enzym ist es auch möglich, die Reaktion in einem Wirbelbett durchzuführen.

Die Reaktionsmischung kann kontinuierlich durch die Säule gepumpt werden, wobei mit der Fließgeschwindigkeit die Verweilzeit und damit der gewünschte Umsatz zu steuern ist. Es ist auch möglich, die Reaktionsmischung im Kreislauf durch die Säule zu pumpen, wobei auch unter Vakuum das Reaktionswasser bzw. -alkanol gleichzeitig abdestilliert werden kann.

Andere Methoden zur Entfernung des Reaktionswassers bzw. -alkanols können auch verwendet werden, z. B. Absorption oder Pervaporation.

### Beispiele

1. 408 g eines polyoxyalkylenmodifizierten Siloxans der Formel

   HO-[(CH(CH₃)CH₂O)(C₂H₄O)_{3,8}]₄-(C₃H₆)-Si(CH₃)₂O- (Si(CH₃)₂O)₁₈-Si(CH₃)₂-(C₃H₆)-[(OC₂H₄)_{3,8}(OCH₂CH(CH₃))]₄-OH

   wurden mit 123 g Butylacrylat und 10,8 g des Enzyms Novozym® 435 gemischt und auf 70 °C erwärmt. Unter Vakuum (20 - 40 mbar) wurde freigesetztes Butanol abdestilliert. Nach 8 h Reaktionszeit wurde der Katalysator abfiltriert und der Rest an Butylacrylat bei 100 - 110 °C abdestilliert. 65 % der Hydroxygruppen wurden acryliert (Nachweis mittels Hydroxyzahl).
   Das so hergestellte Produkt wies in seinen anwendungstechnischen Eigenschaften als Additiv für strahlenhärtende Beschichtungen in Summe mindestens gleich gute Eigenschaften auf wie ein herkömmlich hergestelltes Produkt, jedoch mit dem Vorteil einer deutlich geringeren Eigenfärbung.
2. 120 g polyoxyalkylenmodifiziertes Siloxan gemäß Beispiel 1 wurden mit 180 g Butylacrylat gemischt, auf 70 °C erwärmt und durch eine Säule, die mit 2 g Novozym® 435 gefüllt war, mit einer Fließgeschwindigkeit von 0,64 g/min durchgepumpt. Nach 4 h wurde gebildetes Butanol und der Überschuß Butylacrylat vom gesammelten Produkt abdestilliert. Man erhielt ein Produkt mit einem Acrylierungsgrad von 57 % (Nachweis mittels Hydroxyzahl).

## Patentansprüche

1. Verfahren zur Ver- oder Umesterung von Acrylsäure und/oder Methacrylsäure oder Acrylsäureestern und/oder Methacrylsäureestern in Anwesenheit eines die Ver- oder Umesterung katalysierenden Enzyms mit hydroxyfunktionellen und/oder polyoxyalkylenmodifizierten Siloxanderivaten der allgemeinen Formel (I) 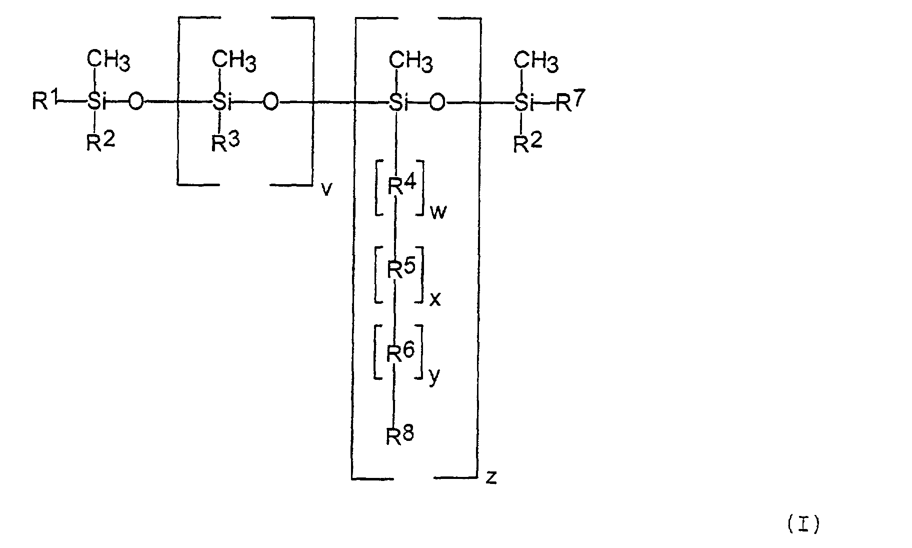 wobei
R¹ und/oder R⁷ = R² oder [R⁴]_{w}― [R⁵]ₓ― [R⁶]_{y}― R⁸,
R² = R³ oder ≠ R³ für gleiche oder verschiedene Alkylreste oder Alkylenreste mit 1 bis 24 Kohlenstoffatomen oder gegebenenfalls substituierte Phenylreste mit bis zu 24 Kohlenstoffatomen steht,
R⁴ = ein zweiwertiger Rest der Formel O, NH, NR², S oder ein Rest der Formel (OSi(CH₃)₂)ᵤ, wobei
u = 1 bis 200,
R⁵ = gleiche oder verschiedene Alkylreste oder Alkylenreste mit 1 bis 24 Kohlenstoffatomen, oder CₙH_{2n-f}R²_{f}― R⁴― CₘH_{2m-g}R²_{g}, wobei
f = 0 bis 12,
g = 0 bis 12,
n = 1 bis 18,
m = 1 bis 18,
R⁶ = O- (C₂H₄₋ₐR²ₐO)_{b}(C_{c}H_{2c}O)_{d}, wobei
a = 0 bis 3,
b = 0 bis 100,
c = 2 bis 12,
d = 0 bis 100,
die Summe (b + d) = 1 bis 200 ist
und die Reihenfolge der einzelnen Polyoxyalkylensegmente (C₂H₄₋ₐR²ₐO)_{b} und (C_{c}H_{2c}O)_{d} beliebig sein kann und insbesondere Blockcopolymere, wie statistische Polymere sowie deren Kombinationen, umfaßt, oder
R⁶ = Oₑ-CₕH₂ₕ-CᵢH₂ᵢ₋ⱼR⁹ⱼ, wobei
e = 0 oder 1,
h = 0 bis 24,
i = 0 bis 24,
j = 1 bis 3,
die Summe (w + e) = 0 bis 1 ist
und R⁹ jeweils ein zweiwertiger Rest der Formel O, eine Hydroxygruppe, ein Rest der Formel CₕH₂ₕ oder ein Rest der Formel CₖH₂ₖ₋₁(OH)₁ bedeutet, wobei
k = 0 bis 24 und
1 = 1 bis 3 ist,
R⁸ = ein Wasserstoffrest oder ein einwertiger organischer Rest ist, wenn y gleich 1 ist, wobei pro Molekül mindestens ein Wasserstoffrest vorhanden sein muß, oder eine OH-Gruppe oder ein einwertiger organischer Rest, wenn y = 0, wobei pro Molekül mindestens eine OH-Gruppe vorhanden ist,
v = 0 bis 200,
w = 0 oder 1,
x = 0 oder 1,
y = 0 oder 1,
z = 0 bis 200
und die Summe (w + x + y) = 1 bis 3
und wenn z = ist, R¹ und/oder R⁷ gleich
[R⁴]_{w}― [R⁵]ₓ― [R⁶]_{y}― R⁸ ist
und wenn x = 0 dann auch w = 0 ist.

2. Verfahren nach Anspruch 1, **dadurch gekennzeichnet, daß** man als Enzym Hydrolasen insbesondere in immobilisierter Form einsetzt, die ausgewählt sind aus Lipasen, Esterasen oder Proteasen, die derartige Ver- und Umesterungsreaktionen katalysieren.

3. Verfahren nach Anspruch 1, **dadurch gekennzeichnet, daß** man die Reaktion bei einer Temperatur im Bereich von 20 bis 100 °C, insbesondere 40 bis 70 °C durchführt.

4. Verfahren nach Anspruch 1, **dadurch gekennzeichnet, daß** man als Acryl- und/oder Methacrylsäuredonor Methyl-, Ethyl-, Propyl- oder Butylester der Acryl- und/oder Methacrylsäure einsetzt.

5. Verfahren nach Anspruch 1, **dadurch gekennzeichnet, daß** 5 bis 100 % der Hydroxygruppen acryliert und/oder methacryliert werden.

6. Verwendung von Acrylaten und/oder Methacrylaten von hydroxyfunktionellen und/oder polyoxyalkylenmodifizierten Siloxanderivaten, erhältlich nach einem Verfahren gemäß einem der Ansprüche 1 bis 5 als Haupt- oder Nebenbestandteil zur Herstellung und/oder Stabilisierung von Dispersionen.

7. Verwendung von Acrylaten und/oder Methacrylaten von hydroxyfunktionellen und/oder polyoxyalkylenmodifizierten Siloxanderivaten, erhältlich nach einem Verfahren gemäß einem der Ansprüche 1 bis 5 als Haupt- oder Nebenbestandteil in strahlenhärtenden Beschichtungen.

8. Verwendung nach Anspruch 7 in transparenten Klarlacken.

9. Verwendung von Acrylaten und/oder Methacrylaten von hydroxyfunktionellen und/oder polyoxyalkylenmodifizierten Siloxanderivaten, erhältlich nach einem Verfahren gemäß einem der Ansprüche 1 bis 5 als Haupt- oder Nebenbestandteil zur Herstellung von Polymeren mittels radikalischer Polymerisation.

## Claims

1. Process for esterifying or transesterifying acrylic acid and/or methacrylic acid or acrylic esters and/or methacrylic esters with hydroxyfunctional and/or polyoxyalkylene-modified siloxane derivatives of the general formula (I) 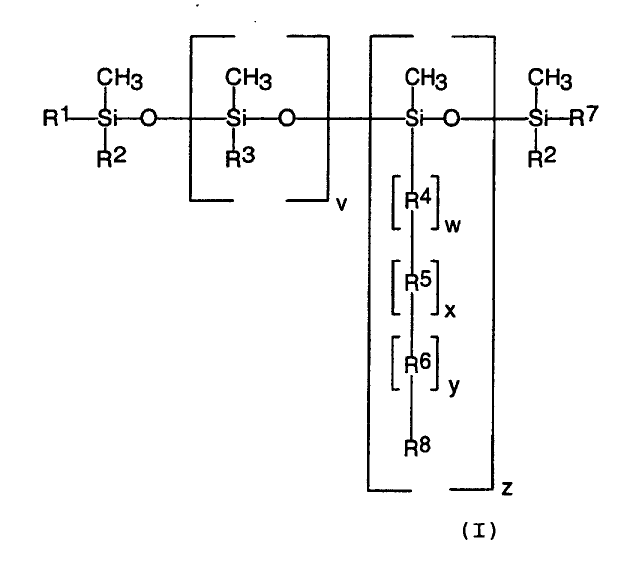 where
R¹ and/or R⁷ = R² or [R⁴]_{w}-[R⁵]ₓ-[R⁶]_{y}-R⁸,
R² = R³ or ≠ R³ for identical or different alkyl radicals or alkylene radicals having 1 to 24 carbon atoms or unsubstituted or substituted phenyl radicals having up to 24 carbon atoms,
R⁴ = a divalent radical of the formula O, NH, NR², S or a radical of the formula (OSi(CH₃)₂)ᵤ, where
u = 1 to 200,
R⁵ = identical or different alkyl radicals or alkylene radicals having 1 to 24 carbon atoms, or CₙH_{2n-f}R²_{f}-R⁴-CₘH_{2m-g}R²_{g}, Where
f = 0 to 12,
g = 0 to 12,
n = 1 to 18,
m = 1 to 18,
R⁶ = O-(C₂H₄₋ₐR²ₐO)_{b}(C_{c}H_{2c}O)_{d}, where
a = 0 to 3,
b = 0 to 100,
c = 2 to 12,
d = 0 to 100,
the sum (b + d) = 1 to 200
and the sequence of the individual polyoxyalkylene segments (C₂H₄₋ₐR²ₐO)_{b} and (C_{c}H_{2c}O)_{d} is arbitrary and
in particular embraces block copolymers, such as random polymers and combinations thereof, or
R⁶ = Oₑ-CₕH₂ₕ-CᵢH₂ᵢ₋ⱼR⁹ⱼ, where
e = 0 or 1,
h = 0 to 24,
i = 0 to 24,
j = 1 to 3,
the sum (w + e) = 0 to 1
and R⁹ is in each case a divalent radical of the formula O, a hydroxyl group, a radical of the formula CₕH₂ₕ or a radical of the formula CₖH₂ₖ₋ ₁(OH)₁ where
k = 0 to 24 and
1 = 1 to 3,
R⁸ = a hydrogen or a monovalent organic radical if y is 1, at least one hydrogen necessarily being present per molecule, or an OH group or a monovalent organic radical if y = 0, at least one OH group being present per molecule,
v = 0 to 200,
w = 0 or 1,
x = 0 or 1,
y = 0 or 1,
z = 0 to 200
and the sum (w + x + y) = 1 to 3
and if z = 0 R¹ and/or R⁷ are/is
[R⁴]_{w}-[R⁵]ₓ-[R⁶]_{y}-R⁸
and if x = 0 then w = 0 as well
in the presence of an enzyme which catalyses the esterification or transesterification.

2. Process according to Claim 1, **characterized in that** the enzyme employed comprises hydrolases, especially in immobilized form, which are selected from lipases, esterases or proteases which catalyse such esterification and transesterification reactions.

3. Process according to Claim 1, **characterized in that** the reaction is carried out at a temperature in the range from 20 to 100°C, in particular from 40 to 70°C.

4. Process according to Claim 1, **characterized in that** methyl, ethyl, propyl or butyl esters of acrylic and/or methacrylic acid are employed as acrylic and/or methacrylic acid donor.

5. Process according to Claim 1, **characterized in that** from 5 to 100% of the hydroxyl groups are acrylated and/or methacrylated.

6. Use of acrylates and/or methacrylates of hydroxyfunctional and/or polyoxyalkylene-modified siloxane derivatives obtainable by a process according to any of Claims 1 to 5 as a principal or secondary constituent for the preparation and/or stabilization of dispersions.

7. Use of acrylates and/or methacrylates of hydroxyfunctional and/or polyoxyalkylene-modified siloxane derivatives obtainable by a process according to any of Claims 1 to 5 as a principal or secondary constituent in radiation-curing coatings.

8. Use according to Claim 7 in transparent clearcoats.

9. Use of acrylates and/or methacrylates of hydroxyfunctional and/or polyoxyalkylene-modified siloxane derivatives obtainable by a process according to any of Claims 1 to 5 as a principal or secondary constituent for the preparation of polymers by means of free-radical polymerization.

## Revendications

1. Procédé pour l'estérification ou la transestérification de l'acide acrylique et/ou de l'acide méthacrylique ou d'esters de l'acide acrylique et/ou d'esters de l'acide méthacrylique en présence d'une enzyme catalysant l'estérification ou la transestérification avec des dérivés siloxane à fonctionnalité hydroxy et/ou modifiés par un polyoxyalkylène, de formule générale (I) 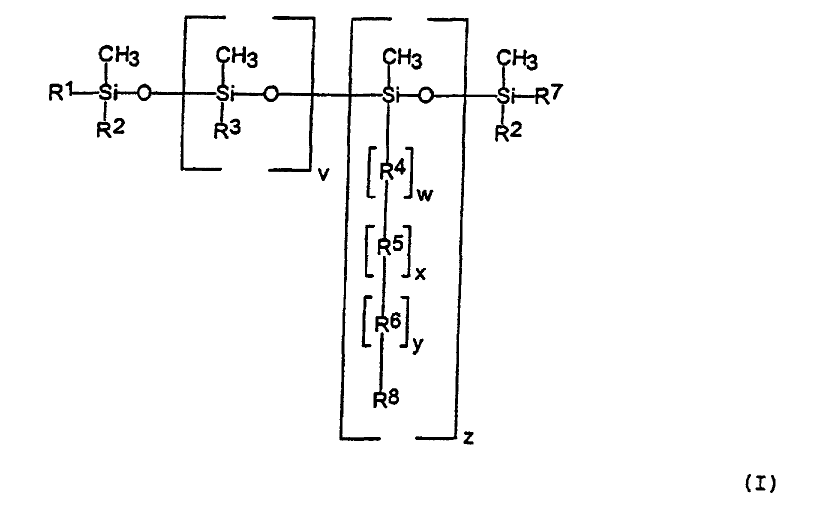 dans laquelle
R¹ et/ou R⁷ = R² ou [R⁴]_{w}-[R⁵]ₓ-[R⁶]_{y}-R⁸,
R² = R³ ou ≠ R³ représente des radicaux alkyle identiques ou différents ou des radicaux alkylène comprenant 1 à 24 atomes de carbone ou des radicaux phényle le cas échéant substitués, comprenant jusqu'à 24 atomes de carbone,
R⁴ = un radical divalent de formule O, NH, NR², S ou un radical de formule (OSi(CH₃)₂)ᵤ, où
u = 1 à 200,
R⁵ = des radicaux alkyle identiques ou différents ou des radicaux alkylène comprenant 1 à 24 atomes de carbone, ou
CₙH_{2n-f}R²_{f}-R⁴-CₘH_{2m-g}R²_{g}, où
f = 0 à 12,
g = 0 à 12,
n = 1 à 18,
m = 1 à 18,
R⁶ = O-(C₂H₄₋ₐR²ₐO)_{b}(C_{c}H_{2c}O)_{d}, où
a = 0 à 3,
b = 0 à 100,
c = 2 à 12,
d = 0 à 100,
la somme (b+d) = 1 à 200
et l'ordre des différents segments de polyoxyalkylène (C₂H₄₋ₐR²ₐO)_{b} et (C_{c}H_{2c}O)_{d} peut être quelconque et comprend en particulier des copolymères à blocs, tels que des polymères statistiques, ainsi que leurs combinaisons ou R⁶ = Oₑ-CₕH₂ₕ-CᵢH₂ᵢ₋ⱼR⁹ⱼ, où
e = 0 ou 1,
h = 0 à 24,
i = 0 à 24,
j = 1 à 3,
la somme (w+e) = 0 à 1
et R⁹ représente à chaque fois un radical divalent de formule 0, un groupement hydroxy, un radical de formule CₕH₂ₕ ou un radical de formule CₖH₂ₖ₋₁(OH)₁ où
k = 0 à 24 et
1 = 1 à 3,
R⁸ = un radical hydrogène ou un radical organique monovalent, lorsque y est égal à 1, au moins un radical hydrogène devant être présent par molécule, ou un groupement OH ou un radical organique monovalent, lorsque y = 0, au moins un groupement OH étant présent par molécule,
v = 0 à 200,
w = 0 ou 1,
x = 0 ou 1,
y = 0 ou 1,
z = 0 à 200,
et la somme (w+x+y) = 1 à 3
et, lorsque z = 0, R¹ et/ou R⁷ sont identiques à
[R⁴]_{w}-[R⁵]ₓ-[R⁶]_{y}-R⁸,
et lorsque x = 0, w est également = 0.

2. Procédé selon la revendication 1, **caractérisé en ce qu'**on utilise comme enzyme des hydrolases en particulier sous forme immobilisée, qui sont choisies parmi les lipases, les estérases ou les protéases qui catalysent de telles réactions d'estérification et de transestérification.

3. Procédé selon la revendication 1, **caractérisé en ce qu'**on effectue la réaction à une température dans la plage de 20 à 100°C, en particulier de 40 à 70°C.

4. Procédé selon la revendication 1, **caractérisé en ce qu'**on utilise comme donneur d'acide acrylique et/ou méthacrylique des esters méthyliques, éthyliques, propyliques ou butyliques de l'acide acrylique et/ou méthacrylique.

5. Procédé selon la revendication 1, **caractérisé en ce que** 5 à 100% des groupements hydroxy sont acrylés et/ou méthacrylés.

6. Utilisation des acrylates et/ou des méthacrylates de dérivés siloxane à fonctionnalité hydroxy et/ou modifiés par un polyoxyalkylène, pouvant être obtenus selon un procédé selon l'une quelconque des revendications 1 à 5 comme constituant principal ou secondaire pour la préparation et/ou la stabilisation de dispersions.

7. Utilisation des acrylates et/ou des méthacrylates de dérivés siloxane à fonctionnalité hydroxy et/ou modifiés par un polyoxyalkylène, pouvant être obtenus selon un procédé selon l'une quelconque des revendications 1 à 5 comme constituant principal ou secondaire dans des revêtements durcissant sous l'effet de rayonnements.

8. Utilisation selon la revendication 7 dans des laques claires transparentes.

9. Utilisation des acrylates et/ou des méthacrylates de dérivés de siloxane à fonctionnalité hydroxy et/ou modifiés par un polyoxyalkylène, pouvant être obtenus selon un procédé selon l'une quelconque des revendications 1 à 5 comme constituant principal ou secondaire pour la préparation de polymères au moyen d'une polymérisation radicalaire.
